# EUROPEAN PATENT SPECIFICATION

(11) **EP 1 838 172 B1**
(45) Date of publication and mention of the grant of the patent: **15.12.2010**
(21) Application number: 06701213.8
(22) Date of filing: 16.01.2006
(51) Int. Cl.: A23L 1/305, A61K 38/01, A61K 31/198, A61P 3/10

(54) **NOVEL NUTRACEUTICAL COMPOSITIONS**
NEUE NUTRAZEUTIKA-ZUSAMMENSETZUNGEN
NOUVELLES COMPOSITIONS NUTRACEUTIQUES

(30) Priority: 18.01.2005 EP 05100271
(43) Date of publication of application: 03.10.2007
(73) Proprietor: DSM IP Assets B.V., 6411 TE Heerlen (NL)
(72) Inventor: WOLFRAM, Swen, 79761 Waldshut-tiengen (DE); LOON VAN, Lucas Johannes Cornelis, NL-6222 VB Maastricht (NL)
(74) Representative: Rabanus, Birgit
(86) International application number: PCT/EP2006/050213
(87) International publication number: WO 2006/077202

(56) References cited:
- WO-A-02/49636
- WO-A-2004/022083
- WO-A-2004/056207
- US-A1- 2004 054 130
- US-A1- 2004 122 097
- US-A1- 2004 192 615
- US-A1- 2004 248 771
- US-B1- 6 436 464
- LOON VAN L J C ET AL: "AMINO ACID INGESTION STRONGLY ENHANCES INSULIN SECRETION IN PATIENTS WITH LONG-TERM TYPE 2 DIABETES" DIABETES CARE, AMERICAN DIABETES ASSOCIATION, ALEXANDRIA, VA, US, vol. 26, no. 3, March 2003 (2003-03), pages 625-630, XP009019276 ISSN: 0149-5992
- VAN LOON LJC, SARIS WHM, VERHAGEN H, WAGENMAKERS AJM: "Plasma insulin responses after ingestion of different amino acid or protein mixtures with carbohydrate" AM. J. CLIN. NUTR., vol. 72, 2000, pages 96-105, XP002321922
- VAN LOON LJC, KRUIJSHOOP M, VERHAGEN H, SARIS WHM, WAGENMAKERS AJM: "Ingestion of protein hydrolysate and amino acid-carbohydrate mixtures increases postexercise plasma insulin responses in men" J. NUTR., vol. 130, 2000, pages 2508-2513, XP002321923

## Description

The present invention relates to a novel nutraceutical composition.

Diabetes mellitus is a widespread chronic disease that hitherto has no cure. The incidence and prevalence of diabetes mellitus is increasing exponentially and it is among the most common metabolic disorders in developed and developing countries. Diabetes mellitus is a complex disease derived from multiple causative factors and characterized by impaired carbohydrate, protein and fat metabolism associated with a deficiency in insulin secretion and/or insulin resistance. This results in elevated fasting and postprandial serum glucose concentrations that lead to complications if left untreated. There are two major categories of the disease, insulin-dependent diabetes mellitus (IDDM, T1DM) and non-insulin-dependent diabetes mellitus (NIDDM, T2DM). T1DM = type 1 diabetes mellitus. T2DM = type 2 diabetes mellitus.

T1DM and T2DM diabetes are associated with hyperglycemia, hypercholesterolemia and hyperlipidemia. The absolute insulin deficiency and insensitivity to insulin in T1DM and T2DM, respectively, leads to a decrease in glucose utilization by the liver, muscle and the adipose tissue and to an increase in the blood glucose levels. Uncontrolled hyperglycemia is associated with increased and premature mortality due to an increased risk for microvascular and macrovascular diseases, including nephropathy, neuropathy, retinopathy, hypertension, stroke, and heart disease. Recent evidence showed that tight glycemic control is a major factor in the prevention of these complications in both T1DM and T2DM. Therefore, optimal glycemic control by drugs or therapeutic regimens is an important approach for the treatment of diabetes.

Therapy of T2DM initially involves dietary and lifestyle changes, when these measures fail to maintain adequate glycemic control the patients are treated with oral hypoglycemic agents and/or exogenous insulin. The current oral pharmacological agents for the treatment of T2DM include those that potentate insulin secretion (sulphonylurea agents), those that improve the action of insulin in the liver (biguanide agents), insulin-sensitizing agents (thiazolidinediones) and agents which act to inhibit the uptake of glucose (α-glucosidase inhibitors). However, currently available agents generally fail to maintain adequate glycemic control in the long term due to progressive deterioration of hyperglycemia, resulting from progressive loss of pancreatic cell function. The proportion of patients able to maintain target glycemia levels decreases markedly over time necessitating the administration of additional/alternative pharmacological agents. Furthermore, the drugs may have unwanted side effects and are associated with high primary and secondary failure rates. Finally, the use of hypoglycemic drugs may be effective in controlling blood glucose levels, but may not prevent all the complications of diabetes. Thus, current methods of treatment for all types of diabetes mellitus fail to achieve the ideals of normoglycemia and the prevention of diabetic complications. L.J.C. van Loon et al, Diabetes Care, Vol. 26, No. 3 March 2003, 625-630, discloses the use of a composition comprising amino acids and protein which composition tripled the insulin response following carbohydrate intake. In this article leucine and phenylalanine are used in this composition in a ratio of 1:1 (see for example the paragraph "beverages" on .page 626).

WO 2004/022083 relates to a nutritional and therapeutic composition of an insulin sensitizer and a peptide fraction. This document discloses the use of an amino acid mixture for a high insulin response. On page 7 lines 12-15, Examples 1 and 4 the free amino acids leucine and phenylalanine are supplemented in equal amounts.

L.J.C. van Loon et al, Am. J. Clin Nutr. Vol. 72, 2000, 96-105 relates to plasma insulin responses after ingestion of different amino acid or protein mixtures with carbohydrate. Table 1 shows that leucine and phenylalanine are used in equal amounts.

L.J.C. van Loon et al, J. Nutr. Vol. 130, 2000, 2508-2513 discloses the ingestion of protein hydrolysate and amino acid-carbohydrate mixtures to increase postexercise plasma insulin responses in men. In all methods used, leucine and phenylalanine is present.

WO 2002/49636 discloses the use of lysine, leucine, cysteine, glycine and glutamic acid as antidiabetic composition.

WO 2004/056207 discloses a blood glucose regulating composition based on a whey hydrolysate.

Therefore, although the therapies of choice in the treatment of T1DM and T2DM are based essentially on the administration of insulin and of oral hypoglycemic drugs, there is a need for a safe and effective nutritional supplement with minimal side effects for the treatment and prevention of diabetes. Many patients are interested in alternative therapies which could minimize the side effects associated with high-dose of drugs and yield additive clinical benefits. Patients with diabetes mellitus have a special interest in treatment considered as "natural" with mild anti-diabetic effects and without major side effects, which can be used as adjuvant treatment. T2DM is a progressive and chronic disease, which usually is not recognize until significant damage has occurred to the pancreatic cells responsible for producing insulin (β-cells of islets of Langerhans). Therefore, there is an increasing interest in the development of a dietary supplement that may be used to prevent β-cell damage and thus, the progression to overt T2DM in people at risk especially in elderly who are at high risk for developing T2DM. Protection of pancreatic β-cells may be achieved by decreasing blood glucose and/or lipid levels as glucose and lipids exert damaging effects on β-cells. The reduction of blood glucose levels can be achieved via different mechanisms, for example by enhancing insulin sensitivity and/or by reducing hepatic glucose production. The reduction of blood lipid levels can also be achieved via different mechanisms, for example by enhancing lipid oxidation and/or lipid storage. Another possible strategy to protect pancreatic β-cells would be to decrease oxidative stress. Oxidative stress also causes β-cell damage with subsequent loss of insulin secretion and progression to overt T2DM.

Therefore, T2DM is a complicated disease resulting from coexisting defects at multiple organ sites: resistance to insulin action in muscle and adipose tissues, defective pancreatic insulin secretion, unrestrained hepatic glucose production. Those defects are often associated with lipid abnormalities and endothelial dysfunction. Given the multiple pathophysiological lesions in T2DM, combination therapy is an attractive approach to its management.

The present invention is defined by the claims and relates to novel nutraceutical compositions comprising protein hydrolysates and leucine and a carbohydrate, wherein at least 70% of the aminoacids present is leucine. The nutraceutical compositions comprising leucine can also comprise unhydrolysed proteins and carbohydrates as the active ingredients for the treatment or prevention of diabetes mellitus, or other conditions associated with impaired glucose tolerance such as syndrome X and obesity. In another aspect the present invention relates to the use of such compositions as a nutritional supplement for the said treatment or prevention, e.g., as an additive to a multi-vitamin preparations comprising vitamins and minerals which are essential for the maintenance of normal metabolic function but are not synthesized in the body. In still another aspect, the invention relates to a method for the treatment of both type 1 and 2 diabetes mellitus and for the prevention of T2DM in those individuals with pre-diabetes, or impaired glucose tolerance (IGT) or obesity which comprises administering to a subject in need of such treatment leucine and protein hydrolysates or unhydrolysed proteins and/or carbohydrates.

The compositions of the present invention are particularly intended for the treatment of both T1DM and T2DM, and for the prevention of T2DM in those individuals with pre-diabetes, or impaired glucose tolerance (IGT), or obesity.

The present invention relates to a composition which comprises leucine and a protein hydrolysate and a carbohydrate, wherein at least 70% of the aminoacids present is leucine. The amino acid leucine is present in the composition or in the ingredients which are intended for use according to the present invention in at least 70 wt%, preferably at least 80wt%, more preferably at least 90wt% of the amino acids present, and less than 30 wt%, preferably less than 20 wt%, more preferably less than 10 wt% of other amino acids are present. This combination of leucine, and protein hydrolysate is advantageously used to increase plasma insulin in blood, preferably for type 2 diabetes or pre-diabetes.

Surprisingly, it is found that this one amino acid combined with the hydrolysate can be used for type 2 diabetes or prediabetes, preferably the lower post-prandial glucose concentrations or to increase post-prandial insulin secretion in blood. Surprisingly, it has been found that using the combination of leucine and hydrolysate gives equal or even better results than for example the combination of two amino acids, such as leucine and phenylalanine and a hydrolysate.

The compositions comprising a combination of active ingredients, i.e. leucine and protein hydrolysates or unhydrolysed proteins and/or carbohydrates synergistically stimulate insulin secretion and increase glucose disposal to insulin sensitive target tissues such as adipose tissue, skeletal muscle and liver and, thus, provide synergistic effects in the treatment of diabetes mellitus.

The term nutraceutical as used herein denotes the usefulness in both the nutritional and pharmaceutical field of application. Thus, the novel nutraceutical compositions can find use as supplement to food and beverages, and as pharmaceutical formulations for enteral or parenteral applications, which may be solid formulations such as capsules or tablets, or liquid formulations, such as solutions or suspensions. As will be evident from the foregoing, the term nutraceutical composition also comprises food and beverages containing leucine and protein hydrolysates or unhydrolysed proteins and/or carbohydrates as well as supplement compositions containing the aforesaid active ingredients.

Protein hydrolysates can be prepared by incubating a protein source with a single protease or a combination of proteases. Such proteases may be any type of protease including but not limited to endo-proteases, amino peptidases, carboxypeptidases or di- and tri-aminopeptidases.

The protein source can in principle be any protein source. A preferred source is casein or whey protein. A composition comprising whey protein according to the invention may be any composition comprising whey protein such as milk, cream and cheese whey. Whey protein preparations are commercially available in several forms such as whey protein concentrates (WPC) and whey protein isolates (WPI). Suitable protein substrates for hydrolysis also include whole milk, skimmed milk, acid casein, rennet casein, acid whey products or cheese whey products. Moreover, vegetable substrates like wheat gluten, milled barley and protein fractions obtained from, for example, soy, rice or corn are suitable substrates.

Protein hydrolysates can be prepared by contacting the protein substrate with one proteolytic enzyme or a combination of proteolytic enzymes. In case more than one protease is used, these proteases can be added to the protein substrate simultaneously. Alternatively, the proteases can be added to the protein in a predefined sequence. Optionally, the addition of the next protease is preceded by an inactivation of the protease or proteases that were used earlier in the hydrolysis process. Such inactivation may be achieved in various ways and the method of choice depends on the protease that has to be inactivated. Inactivation treatments include but are not limited to heat treatment and a change in pH. Alternatively, commercially available hydrolysates can be used.

The degree of hydrolysis (DH) of a protein substrate is an important parameter. The DH that can be achieved for protein hydrolysate and depends on a large number of parameters, which include but are not limited to the choice for a particular protease, the time that is allowed for the hydrolysis to proceed, the reaction conditions (pH, temperature, salt concentration etc) and the pre-treatment of the protein substrate before it is subjected to hydrolysis by the protease. The DH of the hydrolysate suitable for the process according to the invention may range form 5-50, preferably from 10-40, more preferably form 15-35. The hydrolysate may contain free amino acids. Methods to determine the DH are known to the experts in the field, e.g. the OPA-method described by Church et al. (Anal Biochem (1985) 146, 343).

The hydrolysates can be further processed in various ways, methods including but not limited to spray drying, ultrafiltration, freeze drying, vacuum drying. After drying, the dry material may be grinded and/or sieved in order to obtain fractions of a particular particle size range. Compounds may be added to the hydrolysate to facilitate drying or to influence the final characteristics of the dried hydrolysate such as its tendency to form lumps or its wettability.

It has surprisingly been found that a composition comprising leucine and protein hydrolysates or leucine and unhydrolysed proteins or leucine, protein hydrolysates and carbohydrates or leucine, unhydrolysed proteins and carbohydrates synergistically stimulate pancreatic insulin secretion and enhance glucose disposal to insulin sensitive target tissues. The effects of the compositions are much greater than the expected effects estimated by addition of the effects exerted by leucine or protein hydrolysates or unhydrolysed proteins or carbohydrates alone. Thus, compositions comprising leucine and protein hydrolysates or unhydrolysed proteins and/or carbohydrates synergistically increases pancreatic insulin secretion and enhances glucose disposal to insulin sensitive target tissues. Therefore, compositions comprising leucine and protein hydrolysates or unhydrolysed proteins and/or carbohydrates can be used to prevent or treat both T1DM and T2DM, and for the prevention of T2DM in those individuals with pre-diabetes, impaired glucose tolerance (IGT), or obesity.

The use of combinations of leucine and protein hydrolysates or unhydrolysed proteins and/or carbohydrates, which individually exert different mechanisms of action are effective in achieving and maintaining target blood glucose levels in diabetic patients.

The combinations of the active ingredients identified above have been conceived because of their different actions, to take advantage of synergistic and multiorgan effects. Owing to distinct mechanisms of action of the individual active ingredients the combinations not only improve glycemic control, but also result in lower drug dosing in some settings and minimize adverse effects. Because of their distinct mechanisms and sites of action, the specific combinations of dietary supplements discussed above also take advantage of synergistic effects to achieve a degree of glucose lowering greater than single agents can accomplish. Thus, although the therapies of choice in the therapeutic treatment of T1DM and T2DM is based essentially on the administration of insulin and of oral hypoglycemic drugs, appropriate nutritional therapy is also of major importance for the successful treatment of diabetics.

A multi-vitamin and mineral supplement may be added to the nutraceutical compositions of the present invention to obtain an adequate amount of an essential nutrient missing in some diets. The multi-vitamin and mineral supplement may also be useful for disease prevention and protection against nutritional losses and deficiencies due to lifestyle patterns and common inadequate dietary patterns sometimes observed in diabetes. Moreover, oxidant stress has been implicated in the development of insulin resistance. Reactive oxygen species may impair insulin stimulated glucose uptake by disturbing the insulin receptor signalling cascade. The control of oxidant stress with antioxidants such as α-tocopherol (vitamin E) ascorbic acid (vitamin C) may be of value in the treatment of diabetes. Therefore, the intake of a multi-vitamin supplement may be added to the above mentioned active substances to maintain a well balanced nutrition.

Another important use of one amino acid, preferably leucine, and a protein hydrolysate is in increasing the glycogen level for a person in need of increased glycogen level or to rise the insulin secretion for a person in need thereof. The latter uses may be for example for athletes or other persons doing physical exercises. The protein hydrolysate and the leucine, are suitably used as an additve for use in any energy supplementation or metabolic nutrient. The energy supplementation or nutrient can be in the form of beverage, such as sports drinks, energy drinks or other soft drinks, or any other nutrient preparation suitable for an athlete or another person in need of increased glycogen level or increased insulin production. The energy supplementation or nutrient is preferably in a form that it can be orally consumed. This increasing the glycogen level or the rise of the insulin secretion may for example lead to faster rebuilding of glycogen depots and faster rebuilding of degraded muscular proteins.

A sports drink is a beverage which is supposed to rehydrate athletes, as well as restoring electrolytes, sugar, and other nutrients. Sports drinks are usually isotonic, meaning they contain the same proportions of nutrients as found in the human body. (Source: http://en.wikipedia.org/wiki/Sports_drink)

Energy drinks are beverages which contain (legal) stimulants, vitamins (especially B vitamins) and minerals with the intent to give the user a burst of energy. Common ingredients include caffeine, guarana (caffeine from the Guarana plant), taurine, various forms of ginseng, maltodextrin, inositol, carnitine, creatine, glucuronolactone and ginkgo biloba. Some may contain high levels of sugar, or glucose. Many such beverages are flavored and/or colored. (Source: http://en.wikipedia.org/wiki/Energy_drink)

A soft drink is a drink that does not contain alcohol, as opposed to hard drinks, that do. In general, the term is used only for cold beverages. Hot chocolate, tea, and coffee are not considered soft drinks. The term originally referred exclusively to carbonated drinks, and is still commonly used in this manner.(Source: http://en.wikipedia.org/wiki/Soft_drink)

The nutraceutical composition of the present invention contains leucine and protein hydrolysates and a carbohydrate, and at least 70% of the aminoacids present is leucine. Leucine suitably is present in the composition according to the invention in an amount to provide a daily dosage from about 0.001 g per kg body weight to about 1 g per kg body weight of the subject to which it is to be administered. A food or beverage suitably contains about 0.05 g per serving to about 50 g per serving of leucine. If the nutraceutical composition is a pharmaceutical formulation such formulation may contain leucine in an amount from about 0.001 g to about 1 g per dosage unit, e.g., per capsule or tablet, or from about 0.035 g per daily dose to about 70 g per daily dose of a liquid formulation. Protein hydrolysates suitably are present in the composition according to the invention in an amount to provide a daily dosage from about 0.01 g per kg body weight to about 3 g per kg body weight of the subject to which it is to be administered. A food or beverage suitably contains about 0.1 g per serving to about 100 g per serving of protein hydrolysates. If the nutraceutical composition is a pharmaceutical formulation such formulation may contain protein hydrolysates in an amount from about 0.01 g to about 5 g per dosage unit, e.g., per capsule or tablet, or from about 0.7 g per daily dose to about 210 g per daily dose of a liquid formulation.

The composition contains leucine and protein hydrolysates as specified above and carbohydrates. Carbohydrates suitably are present in the composition according to the invention in an amount to provide a daily dosage from about 0.01 g per kg body weight to about 7 g per kg body weight of the subject to which it is to be administered. A food or beverage suitably contains about 0.5 g per serving to about 200 g per serving of carbohydrates. If the nutraceutical composition is a pharmaceutical formulation such formulation may contain carbohydrates in an amount from about 0.05 g to about 10 g per dosage unit, e.g., per capsule or tablet, or from about 0.7 g per daily dose to about 490 g per daily dose of a liquid formulation.

### Dosage ranges (for a 70 kg person)

Leucine: 0.005-70 g/day
Protein hydrolysates: 0.07-210 g/day
Unhydrolysed proteins: 0.07-210 g/day
Carbohydrates: 0.1-490 g/day

### LEGENDS TO THE FIGURES

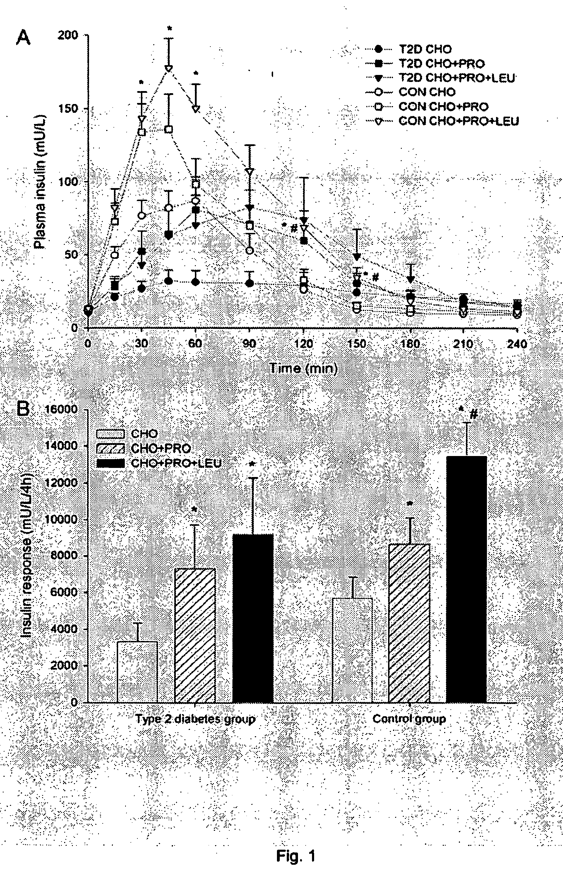

**Figure 1****.** Mean (±SEM) plasma insulin concentrations (A) and response (B) over a 4h period following the ingestion of carbohydrate (CHO; open bars), carbohydrate with a protein hydrolysate (CHO+PRO; hatched bars) and carbohydrate, protein hydrolysate and free leucine (CHO+PRO+LEU; filled bars) in type 2 diabetes patients (T2D) and healthy control subjects (CON). *: significantly different compared to the CHO trial P<0.05, #: significantly different compared to the CHO+PRO trial, P<0.05. No differences were found in insulin responses between groups within the same trial. *n*=10 per group.

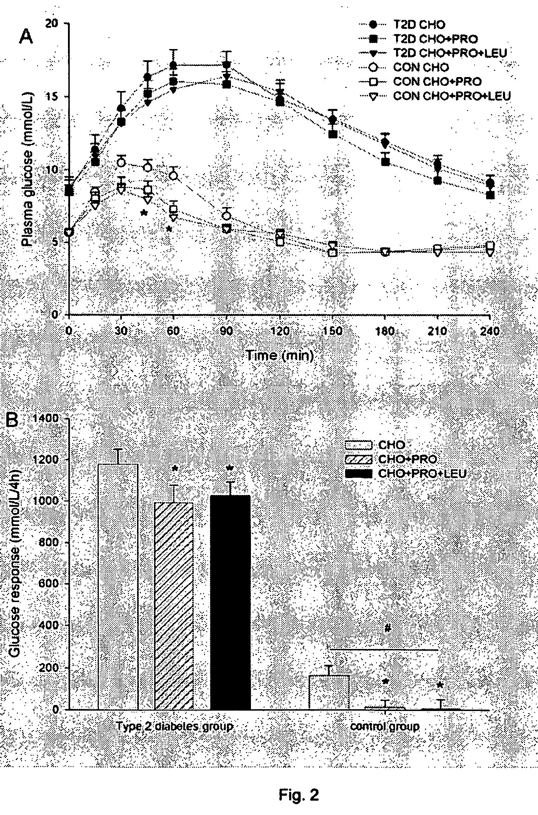

**Figure 2****.** Mean (±SEM) plasma glucose concentrations (A) and response (B) over a 4h period following the ingestion of carbohydrate (CHO; open bars), carbohydrate with a protein hydrolysate (CHO+PRO; hatched bars) and carbohydrate, protein hydrolysate and free leucine (CHO+PRO+LEU; filled bars) in type 2 diabetes patients (T2D) and healthy control subjects (CON). *: significantly different compared to the CHO trial, P<0.05. #: significantly different from diabetes group, P<0.01. *n=*10 per group.

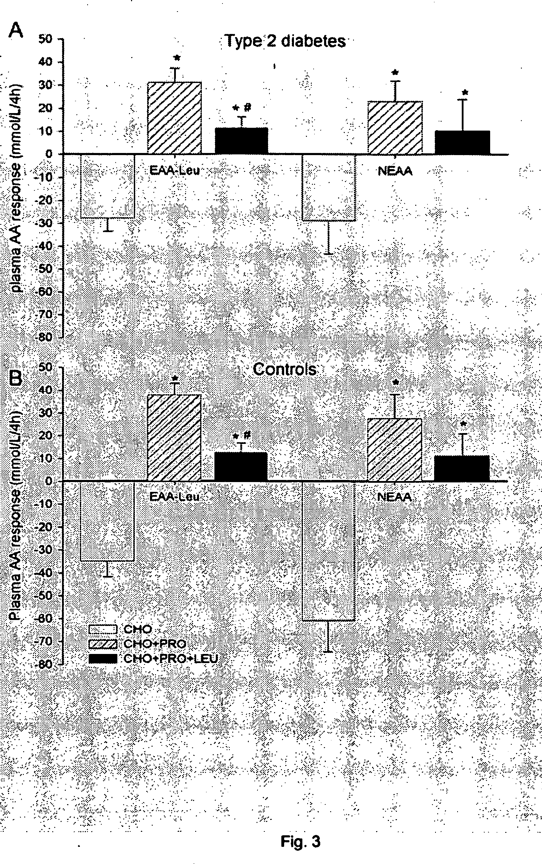

**Figure 3****.** Mean (±SEM) plasma essential (without leucine, EAA-LEU) and non-essential amino acid (NEAA) responses over a 4h period following the ingestion of carbohydrate (CHO), carbohydrate with a protein hydrolysate (CHO+PRO) and carbohydrate, a protein hydrolysate and free leucine (CHO+PRO+LEU) in type 2 diabetes patients (A) and healthy control subjects (B). *: significantly different compared to the CHO trial, P<0.05; #: significantly different compared to the CHO+PRO trial, P<0.05. No differences were found in amino acid responses between groups within the same trial. *n*=10 per group.

The following Examples illustrate the invention further.

Pharmaceutical compositions may be prepared by conventional formulation procedures using the ingredients specified below:

### Example 1 (not according to the invention)

### Soft gelatin capsule

Soft gelatin capsules are prepared by conventional procedures using ingredients specified below:
Active ingredients: Leucine 0.1 g, protein hydrolysates 0.3 g
Other ingredients: glycerol, water, gelatin, vegetable oil

### Example 2 (not according to the invention)

### Hard gelatin capsule

Hard gelatin capsules are prepared by conventional procedures using ingredients specified below:
Active ingredients: Leucine 0.3 g, protein hydrolysates 0.7 g
Other ingredients:
Fillers: lactose or cellulose or cellulose derivatives q.s
Lubricant: magnesium stearate if necessary (0.5%)

### Example 3 (not according to the invention)

### Tablet

Tablets are prepared by conventional procedures using ingredients specified below:
Active ingredients: Leucine 0.4 g, unhydrolysed protein 0.4 g
Other ingredients: microcrystalline cellulose, silicone dioxide (SiO2), magnesium stearate, crosscarmellose sodium.

### B. Food items may be prepared by conventional procedures using ingredients specified below:

### Example 4

### Soft Drink with 30% juice

Typical serving: 240 ml

Active ingredients:
Leucine and protein hydrolysates and maltodextrin as a carbohydrate source are incorporated in this food item:
   Leucine: 0.5-5 g/ per serving
   Protein hydrolysates: 1.5-15 g/ per serving
   Maltodextrin: 3-30 g/ per serving

### I. A Soft Drink Compound is prepared from the following ingredients :

Juice concentrates and water soluble flavors

### 1.1 Orange concentrate

| | **[g]** |
|---|---|
| 60.3 °Brix, 5.15% acidity | 657.99 |
| Lemon concentrate | |
| 43.5 °Brix, 32.7% acidity | 95.96 |
| Orange flavor, water soluble | 13.43 |
| Apricot flavor, water soluble | 6.71 |
| Water 26.46 | |

### 1.2 Color

| | |
|---|---|
| β-Carotene 10% CWS | 0.89 |
| Water | 67.65 |

### 1.3 Acid and Antioxidant

| | |
|---|---|
| Ascorbic acid | 4.11 |
| Citric acid anhydrous | 0.69 |
| Water | 43.18 |

### 1.4 Stabilizers

| | |
|---|---|
| Pectin | 0.20 |
| Sodium benzoate | 2.74 |
| Water | 65.60 |

### 1.5 Oil soluble flavors

| | |
|---|---|
| Orange flavor, oil soluble | 0.34 |
| Orange oil distilled | 0.34 |

### 1.6 Active ingredients

Active ingredients (this means the active ingredient mentioned above: leucine and protein hydrolysates and maltodextrin in the concentrations mentioned above.

Fruit juice concentrates and water soluble flavors are mixed without incorporation of air. The color is dissolved in deionized water. Ascorbic acid and citric acid is dissolved in water. Sodium benzoate is dissolved in water. The pectin is added under stirring and dissolved while boiling. The solution is cooled down. Orange oil and oil soluble flavors are premixed. The active ingredients as mentioned under 1.6 are dry mixed and then stirred preferably into the fruit juice concentrate mixture (1.1).

In order to prepare the soft drink compound all parts 3.1.1 to 3.1.6 are mixed together before homogenizing using a Turrax and then a high-pressure homogenizer (p₁ = 200 bar, p₂ = 50 bar).

### II. A Bottling Syrup is prepared from the following ingredients:

| | [g] |
|---|---|
| Softdrink compound | 74.50 |
| Water | 50.00 |
| Sugar syrup 60° Brix | 150.00 |

The ingredients of the bottling syrup are mixed together. The bottling syrup is diluted with water to 1 l of ready to drink beverage.

### Variations:

Instead of using sodium benzoate, the beverage may be pasteurized. The beverage may also be carbonized.

### Example 5 (not according to the invention)

### Five Cereal Bread

Typical serving: 50 g
Active ingredients: Leucine and unhydrolysed protein and carbohydrates (in the form of five cereal flour) are incorporated in this food item:
Leucine: 0.5-5 g/ per serving
Unhydrolysed proteins: 1.5-15 g/ per serving

| Other components: | [%] |
|---|---|
| Five cereal flour (carbohydrate source) | 56.8 |
| Water | 39.8 |
| Yeast | 2.3 |
| Salt | 1.1 |

The yeast is dissolved in a part of the water. All ingredients are mixed together to form a dough. Salt is added at the end of the kneading time. After fermentation, the dough is reworked and divided before a loaf is formed. Before baking, the surface of the loaf is brushed with water and sprinkled with flour.

### Procedure:

### Kneading:

| | |
|---|---|
| Spiral kneading system | 4 min 1^{st} gear,5 min 2^{nd} gear |
| Dough proofing: | 60 min |
| Dough temperature: | 22 - 24 °C |
| Proofing time: | 30 min |

### Baking:

| | |
|---|---|
| Oven: | Dutch type oven |
| Baking temperature: | 250/220 °C |
| Baking time: | 50 - 60 min |

### Example 6

### Cookies Type Milano

Typical serving: 30 g
Active ingredients: Leucine and protein hydrolysates and carbohydrates (in the form of wheat flour, type 550) are incorporated in this food item:
Leucine: 0.3-3 g/ per serving
Protein hydrolysates: 0.9-9 g/ per serving

| Other components: | [g] |
|---|---|
| Wheat Flour, type 550 (carbohydrate source) | 41.0 |
| Sugar | 20.5 |
| Fat/Butter | 20.5 |
| Whole egg (liquid) | 18.0 |
| Lemon Flavor | q.s. |
| Baking agent | q.s. |

All ingredients are added slowly under mixing to form a sweet short pastry.

Afterwards, the pastry is kept cool (4°C) for at least 2 hours before flattening the pastry to a thickness of approx. 5 mm. Pieces are cut out and brushed with egg yolk on the surface before baking.

### Baking:

Oven: fan oven
Baking temperature: 180 °C
Baking time: 15 min

### Example 7

### Toast

Typical serving: 100 g
Active ingredients:
Leucine and unhydrolysed proteins and carbohydrates (in the form of wheat flour, type 550) are incorporated in this food item:
Leucine: 0.6-6 g/ per serving
Protein hydrolysates: 1.8-18 g/ per serving

| Other components: | [%] |
|---|---|
| Wheat Flour, type 550 (carbohydrate source) | 55.4 |
| Water | 33.2 |
| Yeast | 2.8 |
| Salt | 1.1 |
| Fat/Butter | 5.5 |
| Malt | 0.6 |
| Emulsifier baking agent | 1.4 |

The yeast is dissolved in a part of the water. All ingredients are mixed together to form a dough. Salt is added at the end of the kneading time. Afterwards, the dough is reworked, divided and placed in a baking tin for fermentation. After baking, the loaf is unmoulded directly.

### Procedure:

### Kneading:

Spiral kneading system 5 - 6 min 1^{st} gear; 3 - 4 min 2^{nd} gear
Dough proofing: none
Dough temperature: 22 - 24 °C
Proofing time: 40 min

### Baking:

Oven: Dutch type oven
Baking temperature: 220 °C
Baking time: 35 - 40 min

### Example 8

Yoghurt - set type; 3.5% fat
Typical serving: 225 g
Active ingredients:
Leucine and protein hydrolysates and carbohydrates (in the form of sugar) are incorporated in this food item:
Leucine: 0.5-5 g/ per serving
Protein hydrolysates: 1.5-15 g/ per serving

| Other components: | [%] |
|---|---|
| Full fat milk (3.8% fat) | 90.5 |
| Skimmed milk powder | 2.0 |
| Sugar (carbohydrate source) | 5.0 |
| Culture | 2.5 |

The milk is heated to 35 °C before addition of milk powder, stabilizer, sugar and active ingredients. This mixture is heated to 65 °C to dissolve all ingredients. Then the mixture is homogenized in a high-pressure homogenizer (p₁ = 150 bar, p₂ = 50 bar) at 65 °C. This emulsion is then pasteurized at 80 °C for 20 minutes. After cooling to 45 °C natural yoghurt/culture is added and mixed. Then this mixture is filled into cups and fermented at 45 °C for 3-4 hours until a pH of 4.3 is reached and then stored at 4 °C.

### Example 9

Yoghurt - stirred type; 3.5% fat
Typical serving: 225 g
Leucine and protein hydrolysates and carbohydrates (in the form of sugar) are incorporated in this food item:
Leucine: 0.1-1 g/ per serving
Protein hydrolysates: 0.3-3 g/ per serving

| Other components: | [%] |
|---|---|
| Full fat milk (3.8% fat) | 90.2 |
| Skimmed milk powder | 2.0 |
| Stabilizer | 0.3 |
| Sugar (carbohydrate source) | 5.0 |
| Culture | 2.5 |

The milk is heated to 35 °C before addition of milk powder, stabilizer, sugar and active ingredients. This mixture is heated to 65 °C to dissolve all ingredients before homogenization in a high-pressure homogenizer (p₁ = 150 bar, p₂ = 50 bar) at 65 °C. This emulsion is then pasteurized at 80 °C for 20 minutes. After cooling to 45 °C natural yoghurt/culture is added and mixed, followed by fermentation at 45 °C for 3-4 hours until a pH of 4.3 is reached. After cooling and stirring vigorously, the yoghurt is filled in cups and stored at 4 °C.

### Example 10

Ice cream; 8% fat
Typical serving: 85 g
Active ingredients: Leucine and protein hydrolysates and carbohydrates (in the form of sugar and glucose syrup) are incorporated in this food item:
Leucine: 0.1-1 g/ per serving
Protein hydrolysates: 0.3-3 g/ per serving

| Other components: | [g] |
|---|---|
| Milk (3.7% fat) | 600.00 |
| Cream (35% fat) | 166.00 |
| Skim milk powder | 49.10 |
| Sugar (carbohydrate source) | 109.00 |
| Glucose syrup 80% (carbohydrate source) | 70.00 |
| Ice cream stabilizer | 5.00 |
| Flavor | q.s. |
| Color | q.s |

Sugar, skim milk powder and stabilizer are added to the milk and cream, mixed and heated to 45 °C. Then the color as stock solution and the glucose syrup is added as well as the active ingredients. The mix, is heated up and pasteurized (20 min, 80 °C). Then a homogenization step takes place. Afterwards the mix is cooled down under constant stirring and the flavor is added at 5°C. The mix maturated at 5 °C during at least 4 h and then passed through an ice cream machine (overrun ca. 100%). The ice cream is filled into cups and stored at -20 to -30 °C.

### Example 11

### Wine gums

Active ingredients: Leucine and protein hydrolysates and carbohydrates in the form of sugar crys. And glucose syrup DE 38) are incorporated in this food item:
Leucine: 0.05-0.5 g/ per 30 g
Protein hydrolysates: 0.15-1.5 g/ per 30 g

| Other components: | [g] |
|---|---|
| Gelatin 200 Bloom | 80.0 |
| Water I | 125.0 |
| Sugar crys. (carbohydrate source) | 290.0 |
| Water II | 120.0 |
| Glucose-syrup DE 38 (carbohydrate source) | 390.0 |
| Citric acid | 10.0 |
| Flavor | 2.0 |
| Color | q.s. |
| Yield ca | 1000.0 |

Disperse gelatin in water I, stir and dissolve by heating over a stream bath or using a microwave. Mix sugar with water II and bring to boiling until a clear solution is obtained. Remove from heat source. Mix with glucose syrup while dissolved sugar solution is still hot. Slowly add the gelatin solution. Let rest until foam on surface can be removed and 60-65°C is reached. Add flavor, citric acid and the color solution as well as active ingredients under stirring. Deposit into moulds printed into starch trays and let sit for at least 48 hours at RT. Remove starch powder and polish with oil or wax. Dry at RT and package into airtight pouches

### Example 12

This example shows the post-prandial plasma insulin and glucose responses following the coingestion of an insulinotropic protein hydrolysate with and without additional leucine combined with a single bolus of carbohydrate. Ten long-term diagnosed male type 2 diabetes patients and ten healthy control subjects participated in 3 trials in which the plasma glucose, insulin and amino acid responses were determined following the ingestion of different beverage compositions (carbohydrate, CHO; carbohydrate with protein hydrolysate, CHO+PRO; or carbohydrate, protein hydrolysate and free leucine, CHO+PRO+LEU). Plasma insulin responses were 141 and 204% greater in the type 2 diabetes patients and 66 and 221% greater in the controls in the CHO+PRO and the CHO+PRO+LEU trial, respectively, when compared to the CHO trial (P<0.05). The concomitant plasma glucose responses were 15 and 12% lower in the type 2 diabetes patients and 92 and 97% lower in the controls, respectively, when compared to the CHO trial (P<0.05). Plasma leucine concentrations correlated strongly with the insulin response (r=0.43, P<0.001). We conclude that co-ingestion of a protein hydrolysate with or without additional free leucine augments the insulin response following carbohydrate ingestion, thereby significantly reducing post-prandial glucose excursions in long-term diagnosed type 2 diabetes patients.

### SUBJECTS AND METHODS

### Subjects

Ten long-term diagnosed, male type 2 diabetes patients and ten healthy, for age and BMI matched, control subjects were selected to participate in this study. Subjects' characteristics are provided in Table 1. Exclusion criteria were impaired renal or liver function, obesity (BMI>35 kg/m2), cardiac disease, hypertension, diabetes complications, and exogenous insulin therapy. All type 2 diabetes patients were using oral plasma glucose lowering medication (metformin and/or sulfonylureas). Blood glucose-lowering medication was withheld for 2 days prior to the screening and sulfonylureas, but not metformin, were withheld 2 days before each of the trials. All subjects were informed about the nature and the risks of the experimental procedures before their written informed consent was obtained. All clinical trials were approved by the local Medical Ethical Committee.

**Table 1. Subjects' characteristics¹**

| | **Controls** | **Type 2 diabetes** |
|---|---|---|
| *n* | 10 | 10 |
| Age (yrs) | 60.2±1.3 | 59.7±2.6 |
| Body weight (kg) | 83.7±3.1 | 83.6±3.4 |
| Height (m) | 1.75±0.01 | 1.77±0.02 |
| BMI (kg/m²) | 27.2±1.00 | 26.8±0.82 |
| Basal plasma Glucose (mmol/L) | 5.8±0.1 | 10.3±0.7* |
| Plasma glucose_{OGTT120} (mmol/L)² | 6.1±0.4 | 19.7±0.8*^{#} |
| Basal plasma insulin (mU/L) | 14.2±1.3 | 14.1±2.7 |
| HbA1c (%) | 5.6±0.1 | 8.1±0.3* |
| OGIS₁₂₀ (ml/min/m²)³ | 351±16 | 258±13* |
| Diagnosed with type 2 diabetes (yrs) | NA | 9.2±1.44 |
| Medication | NA | Metformin and/or SU-derivatives |

| | | |
|---|---|---|
| ¹ Values are expressed as means±SEM ² Plasma glucose concentration after an 2h OGTT ³ Oral glucose insulin sensitivity index for a 2 hour OGTT as described elsewhere (22). **^{*}** Different from control group P<0.01. ^{#} Different from basal values P<0.01. | | |

### Screening

Before selection into the study, all subjects performed an oral glucose tolerance test (OGTT). After an overnight fast, subjects arrived at the laboratory at 8.00 am by car or public transportation. A catheter (Baxter BV, Utrecht, the Netherlands) was inserted into an antecubital vein and a resting blood sample was drawn after which 75 g glucose (dissolved in 250 ml water) was ingested. After the bolus was consumed, blood was sampled every 30 min until t=120 min. Plasma glucose concentrations were measured to determine glucose intolerance and/or type 2 diabetes according to the World Health Organization criteria of 1999 (see: Alberti, K. G. & Zimmet, P. Z. (1998) Definition, diagnosis and classification of diabetes mellitus and its complications. Part 1: diagnosis and classification of diabetes mellitus provisional report of a WHO consultation. Diabet Med 15: 539-553). In addition, plasma glucose and insulin concentrations were used to assess insulin sensitivity using the oral glucose insulin sensitivity (OGIS)-index for a 2 hour OGTT as described by Mari et al J. J. (2001). Diabetes Care 24: 539-548.

### Design

Each subject participated in 3 trials, separated by at least 7 days, in which plasma glucose, insulin and amino acid responses were determined following the ingestion of 3 different beverage compositions (CHO: carbohydrate, CHO+PRO: carbohydrate with a casein protein hydrolysate or CHO+PRO+LEU: carbohydrate, a casein protein hydrolysate and leucine). Subjects were placed in a supine position and remained inactive for a period of 4 hours. Drinks were provided in a randomized order and double blind fashion.

### Protocol

After an overnight fast, subjects reported to the laboratory at 8.00 am by car or public transportation. A Teflon catheter (Baxter BV, Utrecht, the Netherlands) was inserted into an antecubital vein for venous blood sampling and a resting blood sample was collected. At t=0 min subjects drank a single bolus (4 mL/kg) of the experimental beverage. Blood samples were drawn every 15 min during the first hour after which blood was sampled at 30 min intervals until t=240 min for measurement of plasma glucose and insulin concentrations. Plasma amino acid concentrations were determined at 1 hour intervals.

### Diet and activity prior to testing

All subjects maintained their normal dietary and physical activity patterns throughout the entire experimental period. In addition, subjects refrained from heavy physical labor and/or exercise training for at least 3 days prior to each trial and filled out a food intake diary for 2 days prior to the first trial to keep their dietary intake as identical as possible prior to the other trials. The evening before each trial, subjects received a standardized meal (43.80 kJ/kg body weight; consisting of 60 Energy % (En%) carbohydrate, 28 En% fat and 12 En% protein).

### Beverages

The subjects received a single bolus (4 mL/kg) containing 0.7 g/kg carbohydrate (50% glucose and 50% maltodextrin, CHO) with 0.3 g/kg of a casein protein hydrolysate (CHO+PRO) or 0.3 g/kg of a casein protein hydrolysate and 0.1 g/kg of leucine (CHO+PRO+LEU). Glucose and maltodextrin were obtained from AVEBE (Veendam, the Netherlands), crystalline leucine from BUFA (Uitgeest, the Netherlands), and the casein protein hydrolysate was prepared by DSM Food Specialties (Delft, the Netherlands). The casein hydrolysate (Insuvital™) was obtained by enzymatic hydrolysis of sodium caseinate using a neutral protease and a prolyl-specific endoproteinase. Drinks were uniformly flavoured by adding 0.2 g sodiumsaccharinate, 1.8 g citric acid, and 5 g cream vanilla flavor (Quest International, Naarden, the Netherlands) per liter beverage.

### Blood sample analysis

Blood was collected in EDTA containing tubes and centrifuged at 1,000 g and 4°C for 10 min. Aliquots of plasma were immediately frozen in liquid nitrogen and stored at -80°C until analyses. Glucose concentrations (Uni Kit III, Roche, Basel) were analyzed with the COBAS FARA semi-automatic analyzer (Roche). Plasma insulin was determined by radioimmunoassay (HI-14K, Linco research Inc, St. Charles, USA). Free amino acids were analyzed using ion-exchange chromatography (JEOL, AminoTac JLC-500/V) with postcolumn ninhydrin derivatisation with norvaline as an internal standard. Prior to analyses samples were deproteinated with 5-sulphosalicylic acid. To determine HbA1c content a 3 ml blood sample was collected in EDTA containing tubes and analyzed by high-performance liquid chromatography(Bio-Rad Diamat, Munich, Germany).

### Statistics

Data are expressed as means±SEM. The plasma responses were calculated as area under the curve above baseline values. To compare plasma metabolite concentrations over time between trials, a two-way repeated measures analysis of variance (ANOVA) was applied. Changes in time within each group were checked for statistical significance using one-way repeatedmeasures ANOVA. A Scheffé's post-hoc test was applied in case of a significant F-ratio to locate specific differences. For non-time dependent variables, multiway ANOVA or Student's t-test was applied. Significance was set at the 0.05 level of confidence. All calculations were performed using StatView 5.0 (SAS Institute inc., Cary, NC, USA).

### RESULTS

### Plasma insulin concentrations

Baseline plasma insulin concentrations were similar between groups and trials and averaged 11.2±1.5 and 13.0±1.1 mU/L for the type 2 diabetes and control group respectively (Fig. 1A, P=0.1). After ingestion of the different beverages, plasma insulin concentrations did not increase in the CHO trial and increased in the CHO+PRO and the CHO+PRO+LEU trials in the type 2 diabetes patients (P<0.05). In the control group a strong increase in plasma insulin concentration was observed during the first h after beverage ingestion (P<0.05). This increase was most pronounced in the CHO+PRO and the CHO+PRO+LEU trials. In all trials, plasma insulin concentrations returned to baseline concentrations during the last h of the experimental in both groups. Insulin responses (AUC above baseline values) in the diabetes group were 141±40 and 204±37% greater in the CHO+PRO and the CHO+PRO+LEU trial, respectively, when compared to the CHO trial (P<0.05, Fig 1B). In the control group, insulin responses were 66+20 and 221±82% greater in the CHO+PRO and CHO+PRO+LEU trial, respectively, compared to the CHO trial (P<0.05). Furthermore, in the control group, the insulin response in the CHO+PRO+LEU trial was significantly greater compared to the CHO+PRO trial (P<0.05). No differences were observed in insulin responses between groups within the same trial.

### Plasma glucose concentrations

Fasting plasma glucose concentrations were higher in the type 2 diabetes patients compared to the normoglycemic controls (8.6±0.6 vs. 5.7±0.1 mmol/L, respectively P<0.01). Following ingestion of the different beverages, plasma glucose concentrations remained significantly higher in the diabetes patients compared to their matched controls in all trials (P<0.01, Fig. 2A). In the type 2 diabetes patients, plasma glucose concentrations significantly increased during the first h following beverage ingestion, after which values returned to near baseline values (Fig 2A). Plasma glucose concentrations in the control group increased during the first 30 min after ingestion of the test drinks, after which plasma glucose concentrations also returned to baseline values. In the control group, plasma glucose concentrations decreased faster in the CHO+PRO and the CHO+PRO+LEU trials when compared to the CHO trial, resulting in lower plasma glucose concentrations at t=45 and t=60 min (P<0.05, Fig 2A). After expressing the postprandial glucose response as area under the curve above baseline values (Fig 2B), the glucose responses were reduced by 15±5 and 12±3% in the type 2 diabetes group and by 92±2 and 97±3% in the control group in the CHO+PRO and CHO+PRO+LEU trials, respectively, when compared to the CHO trial (P<0.05). Plasma glucose responses were substantially higher in the diabetes patients when compared to the controls in all trials (P<0.01, Fig 2B). Glucose responses were inversely correlated with the accompanying insulin response in the type 2 diabetes patients (r=-0.48, P<0.01).

### Plasma amino acid concentrations

Fasting plasma amino acid concentrations are reported in Table 2.

**Table 2. Basal plasma amino acid concentrations¹**

| | **Controls** | **Type 2 Diabetes** |
|---|---|---|
| 1-Methyl-hystidine | 9.5±1.3 | 9.4±1.4 |
| 3-Methyl-hystidine | 22.3±2.5 | 27.7±2.0 |
| α-Aminobuterate | 29.2±1.1 | 31.5±1.1 |
| Alanine | 370.3±19.2 | 431.1±17.0* |
| Arginine | 128.1±7.0 | 110.2±3.5* |
| Asparagine | 10.0±0.5 | 11.0±0.6 |
| Aspartic-acid | 39.0±1.1 | 34.8±1.2* |
| Citrulline | 48.9±1.6 | 43.7±3.1 |
| Cysteine | 54.1±1.4 | 54.8±1.4 |
| Glutamine | 94.3±4.3 | 109.7±5.9* |
| Glutamic acid | 528.5±7.4 | 508.4±14.4 |
| Glycine | 208.2±8.8 | 207.8±9.5 |
| Histidine^{†} | 71.9±1.7 | 69.0±1.7 |
| Isoleucine^{†} | 66.0±2.0 | 79.1±2.3* |
| Leucine^{†} | 122.8±3.0 | 144.9±3.2* |
| Lysine^{†} | 187.8±4.7 | 204.2±5.4* |
| Methionine^{†} | 21.7±0.6 | 20.6±0.7 |
| Ornithine | 50.4±1.6 | 51.7±1.4 |
| Phenylalanine^{†} | 52.6±1.2 | 50.5±1.0 |
| Proline | 77.1±3.1 | 94.7±5.8* |
| Serine | 92.7±1.7 | 90.1±3.0 |
| Threonine^{†} | 112.0±3.7 | 118.6±4.6 |
| Tryptophan^{†} | 41.3±2.3 | 37.5±1.9 |
| Tyrosine | 62.3±2.9 | 56.8±2.0 |
| Valine^{†} | 216.7±5.0 | 252.4±4.9* |

| | | |
|---|---|---|
| ¹ Values are expressed (in µmol/L) as means±SEM, *n*=10 for type 2 diabetes patients and n=10 for controls ^{†}: Essential amino acid. * Different from control group P<0.05. | | |

At baseline, the plasma essential amino acids (EAAs): leucine (144.9±3.2 vs. 122.8±3.0 µmol/L), isoleucine (79.1±2.3 vs. 66.0±2.0 µmol/L), lysine (204.2±5.4 vs. 187.8±4.7 µmol/L) and valine (252.4±4.9 vs. 216.7±5.0 µmol/L) and the non-essential amino acids (NEAAs): alanine (431±17.0 vs. 370.3±19.2 µmol/L), glutamine (109.7±5.9 vs. 94.3±4.3 µmol/L) and proline (94.7±5.8 vs. 77.1±3.1 µmol/L) concentrations were higher in the type 2 diabetes patients compared to the matched controls (P<0.05). Plasma arginine (110±0.6 vs. 128.1±7.0 µmol/L) and aspartic acid (34.8±1.2 vs. 39.0±1.1µmol/L) concentrations were lower in the basal, fasted state in the type 2 diabetes patients when compared to the control subjects (P<0.05). A complete overview of the subsequent plasma free amino acid responses, calculated as area under curve above baseline values, is provided in Table 3.

**Table 3. Plasma amino acid responses after beverage ingestion¹**

| | **Controls** | | | **Type 2 Diabetes** | | |
|---|---|---|---|---|---|---|
| | **CHO** | **CHO+PRO** | **CHO+PRO +LEU** | **CHO** | **CHO+PRO** | **CHO+PRO +LEU** |
| 1-Methyl-hystidine | -0.2±0.1 | -0.2±0.2 | -0.1±0.1 | -0.5±0.2 | -0.2±0.1 | -0.2±0.2 |
| 3-Methyl-hystidine | -1.1±0.7 | 1.3±0.5⁺ | 0.6±0.6 | -1.8±0.6 | -0.1±0.3* | -0.5±0.6 |
| α-Aminobuterate | -0.6±0.3 | 0.1±0.3 | -0.7±0.1 | -0.5±0.2 | -0.2±0.2 | -0.7±0.3 |
| Alanine | -0.9±5.7 | 13.0±4.9 | 5.8±4.5 | 5.4±5.2 | 11.8±4.8 | 3.7±6.3 |
| Arginine | -3.1±1.3 | 0.4±1.0 | -0.5±3.4 | -2.7±1.8 | 0.3±0.9 | 0.7±1.3 |
| Asparagine | -2.1±0.4 | 1.41±0.3⁺ | 0.2±0.2^{+#} | -0.8±0.3 | 1.2±0.4⁺ | 0.3±0.5 |
| Aspartic-acid | -0.2±0.2 | 0.3±0.1 | -0.4±0.2 | -0.3±0.3 | 0.4±0.2 | -0.2±0.1 |
| Citrulline | -3.8±0.5 | -0.3±0.4⁺ | 1.1±0.2⁺ | -3.4±0.8 | -1.3±0.7 | 0.1±1.0⁺ |
| Cysteine | -0.8±0.3 | -0.8±0.4 | -1.0±0.2 | -0.3±0.3 | -0.6±0.1 | -0.7±0.3 |
| Glutamic acid | -12.5±2.7 | -2.7±1.4⁺ | 3.6±1.7⁺ | -4.0±3.2 | -1.5±2.6 | -0.4±10.0 |
| Glutamine | -3.9±0.9 | 0.0±2.0 | -4.7±1.4 | -1.0±1.5 | 0.2±1.3 | 2.3±1.2 |
| Glycine | -6.1±0.8 | -3.8±0.9 | -6.1±1.0 | -4.0±1.7 | -4.3±1.0 | -7.1±1.8 |
| Histidine^{†} | -1.4±0.6 | 1.0±0.4⁺ | -0.3±0.4 | -1.7±0.5 | 0.2±0.6⁺ | -1.0±0.6 |
| Isoleucine^{†} | -4.2±0.6 | 4.4±0.5⁺ | -1.4±0.8^{+#} | -4.4±0.7 | 4.9±0.9⁺ | 0.7±0.8 |
| Leucine^{†} | -7.5±1.1 | 5.6±0.9⁺ | 81.7±4.3^{+#} | -7.9±1.0 | 5.4±1.3⁺ | 80.3±26.9^{+#} |
| Lysine^{†} | -4.9±0.9 | 11.1±1.2⁺ | 11.4±1.1⁺ | -4.2±1.2 | 8.2±1.9⁺ | 6.8±1.0⁺ |
| Methionine^{†} | -1.5±0.2 | -1.6±0.3⁺ | 0.9±0.3⁺ | -1.0±0.2 | 1.5±0.2⁺ | 0.9±0.3⁺ |
| Ornithine | -2.1±0.2 | 2.0±0.4⁺ | 2.8±0.3⁺ | -2.5±0.4 | 1.7±0.3⁺ | 2.7±0.5⁺ |
| Phenylalanine^{†} | -2.3±0.4 | 1.7±0.4⁺ | 0.5±0.4 | -1.5±0.3 | 1.1±0.2⁺ | 0.8±0.7⁺ |
| Proline | -1.8±0.7 | 9.4±0.9⁺ | 6.7±0.9⁺ | -2.4±0.8 | 9.5±1.1⁺ | 8.0±1.1⁺ |
| Serine | -4.3±0.6 | 2.6±0.6⁺ | -0.2±0.5^{+#} | -1.8±0.5* | 2.6±0.9⁺ | 0.0±1.4 |
| Threonine^{†} | -4.5±0.9 | 4.6±0.8⁺ | 1.3±0.7^{+#} | -3.2±0.9 | 3.9±1.1⁺ | 1.2±1.5⁺ |
| Tryptophan^{†} | -2.3±0.8 | 1.7±0.9⁺ | 0.1±0.7 | -2.1±0.9 | 0.5±0.3⁺ | -1.6±0.7 |
| Tyrosine | -3.3±0.5 | 5.0±0.7⁺ | 4.1±1.3⁺ | -2.6±0.4 | 3.7±0.6⁺ | 3.3±0.7⁺ |
| Valine^{†} | -8.4±0.8 | 11.8±1.5⁺ | 0.2±1.2^{+#} | -7.7±1.4 | 10.9±1.7 + | 3.5±1.3^{+#} |

| | | | | | | |
|---|---|---|---|---|---|---|
| ¹ Values are areas under the curve minus baseline (in mmo/L/4h) and expressd as means±SEM, *n*=10 for type 2 diabetes patients and n=10 for controls ^{†}: Essential amino acid. **^{*}**Different from control group between trials P<0.05. ⁺ Different from CHO trial within groups P<0.05. ^{#}Different from CHO+PRO trial within groups P<0.05. | | | | | | |

Generally, amino acid responses were negative in the CHO trial, positive in the CHO+PRO trial and of an intermediate value after leucine co-ingestion in the CHO+PRO+LEU trial. Strong positive correlations were observed between the insulin response and the increases in plasma leucine (P<0.001), citrulline (P<0.001), cysteine (P<0.04), lysine (P<0.001), methionine (P<0.04), ornithine (P<0.01) and proline (P<0.01). When calculating the responses for the non-essential (NEAA) and essential amino acids response, with the exception of the supplemented leucine (EAALEU), the following responses were observed in the type 2 diabetes (Fig 3A) and control group (Fig 3B). In the type 2 diabetes group, the EAA-LEU response, was negative in the CHO trial and significantly greater in the CHO+PRO and CHO+PRO+LEU trials (-27.7±5.8 vs. 31.2+6.1 and 11.5±4.6 mmol/L/4h, respectively P<0.05). Furthermore, the EAA-LEU response was significantly lower (60±4%, P<0.05) in the CHO+PRO+LEU compared to the CHO+PRO trial. Plasma NEAA responses were negative in the CHO trial and were significantly greater in the CHO+PRO and CHO+PRO+LEU trials in the diabetes patients (-28.8±14.7 vs. 23.1±8.8 and 10.2±13.8 mmol/L/4h, respectively; P<0.05). Similar findings were observed in the control group. A negative plasma EAA-LEU response was observed in the CHO trial, and significantly greater EAA-LEU responses were observed in the CHO+PRO and CHO+PRO+LEU trials (-35.0±6.7 vs. 37.9+5.1 and 12.7±4.1 mmol/L/4h, respectively; P<0.05). Addition of leucine in the CHO+PRO+LEU trial resulted in a 65±5% lower plasma EAA-LEU response when compared to the CHO+PRO trial (P<0.05). Plasma NEAA responses were negative in the CHO trial and were significantly greater in the CHO+PRO and CHO+PRO+LEU trials (-60.8±13.6 vs. 27.6±10.6 and 11.2+9.8 mmol/L/4h, respectively; P<0.05). No differences in amino acid responses were observed between groups.

## Claims

1. A .composition which comprises leucine, a protein hydrolysate and a carbohydrate and wherein at least 70 wt% of the amino acids present is leucine..

2. A composition of claim 1 wherein at least 80 wt% of the amino acids present is leucine.

3. A composition of claim 1 or 2 wherein at least 90 wt% of the amino acids present is leucine.

4. A composition according to any one of the claims 1 to 3 which is suitable for the treatment or prevention of type 2 diabetes mellitus (T2DM) in those individuals with pre-diabetes, or impaired glucose tolerance (IGT), or obesity, or established type 2 diabetes mellitus.

5. A composition according to claim 1 wherein unhydrolysed protein is present.

6. A composition according to anyone of claims 1 to 5, comprising leucine in an amount sufficient to administer to a subject a daily dosage of 0.005 g per kg body weight to about 1 g per kg body weight.

7. A composition according to claims 1 to 6, comprising protein hydrolysates in an amount sufficient to administer to a subject a daily dosage of 0.01 g per kg body weight to about 3 g per kg body weight.

8. A composition according to claim 5, comprising unhydrolysed proteins in an amount sufficient to administer to a subject a daily dosage of 0.01 g per kg body weight to about 3 g per kg body weight.

9. A composition according to claim 1, comprising carbohydrates in an amount sufficient to administer to a subject a daily dosage of 0.01 g per kg body weight to about 7 g per kg body weight.

10. A composition according to any one of the claims 1 to 9 to increase plasma insulin.

11. A composition according to any one of the claims 1 to 9 to increase plasma insulin of type 2 diabetes or pre-diabetes.

12. A composition according to any one of the claims 1 to 9 to lower post-prandial glucose concentrations in blood of type 2 diabetes or pre-diabetes.

13. A composition according to any one of the claims 1 to 9 to increase post-prandial insulin secretion in blood of type 2 diabetes or pre-diabetes.

## Patentansprüche

1. Zusammensetzung, die Leucin, ein Proteinhydrolysat und ein Kohlenhydrat enthält und wobei wenigstens 70 Gew.-% der vorhandenen Aminosäuren Leucin sind.

2. Zusammensetzung nach Anspruch 1, wobei wenigstens 80 Gew.-% der vorhandenen Aminosäuren Leucin sind.

3. Zusammensetzung nach Anspruch 1 oder 2, wobei wenigstens 90 Gew.-% der vorhandenen Aminosäuren Leucin sind.

4. Zusammensetzung nach einem der Ansprüche 1 bis 3, die sich für die Behandlung oder Prävention von Diabetes mellitus vom Typ 2 (Type 2 Diabetes mellitus, T2DM) bei Individuen mit Prädiabetes oder gestörter Glucosetoleranz (Impaired Glucose Tolerance, IGT) oder Obesitas oder etabliertem Diabetes mellitus vom Typ 2 eignet.

5. Zusammensetzung nach Anspruch 1, wobei nichthydrolisiertes Protein vorhanden ist.

6. Zusammensetzung nach einem der Ansprüche 1 bis 5, die Leucin in einer Menge enthält, die ausreicht, um einem Patienten eine Tagesdosis von 0,005 g pro kg Körpergewicht bis etwa 1 g pro kg Körpergewicht zu verabreichen.

7. Zusammensetzung nach einem der Ansprüche 1 bis 6, die Proteinhydrolysate in einer Menge enthält, die ausreicht, um einem Patienten eine Tagesdosis von 0,01 g pro kg Körpergewicht bis etwa 3 g pro kg Körpergewicht zu verabreichen.

8. Zusammensetzung nach Anspruch 5, die nichthydrolisierte Proteine in einer Menge enthält, die ausreicht, um einem Patienten eine Tagesdosis von 0,01 g pro kg Körpergewicht bis etwa 3 g pro kg Körpergewicht zu verabreichen.

9. Zusammensetzung nach Anspruch 1, die Kohlenhydrate in einer Menge enthält, die ausreicht, um einem Patienten eine Tagesdosis von 0,01 g pro kg Körpergewicht bis etwa 7 g pro kg Körpergewicht zu verabreichen.

10. Zusammensetzung nach einem der Ansprüche 1 bis 9 zum Erhöhen des Plasmainsulins.

11. Zusammensetzung nach einem der Ansprüche 1 bis 9 zum Erhöhen des Plasmainsulins bei Diabetes vom Typ 2 oder Prädiabetes.

12. Zusammensetzung nach einem der Ansprüche 1 bis 9 zum Senken der postprandialen Glucosekonzentrationen im Blut bei Diabetes vom Typ 2 oder Prädiabetes.

13. Zusammensetzung nach einem der Ansprüche 1 bis 9 zum Senken der postprandialen Insulinsekretion im Blut bei Diabetes vom Typ 2 oder Prädiabetes.

## Revendications

1. Composition, **caractérisée en ce qu'**elle comprend de la leucine, un hydrolysat de protéine et un glucide et **en ce qu'**au moins 70 % en poids des acides aminés présents sont la leucine.

2. Composition de la revendication 1, **caractérisée en ce qu'**au moins 80 % en poids des acides aminés présents sont la leucine.

3. Composition de la revendication 1 ou 2, **caractérisée en ce qu'**au moins 90 % en poids des acides aminés présents sont la leucine.

4. Composition selon l'une quelconque des revendications 1 à 3, **caractérisée en ce qu'**elle convient au traitement ou à la prévention du diabète sucré de type 2 (T2DM) chez les individus atteints de pré-diabète, ou de tolérance au glucose altérée (IGT), ou d'obésité, ou de diabète sucré de type 2 établi.

5. Composition selon la revendication 1, **caractérisée en ce qu'**une protéine non hydrolysée est présente.

6. Composition selon l'une quelconque des revendications 1 à 5, comprenant de la leucine dans une quantité suffisante pour administrer à un sujet un dosage journalier de 0,005 g par kg de poids corporel à environ 1 g par kg de poids corporel.

7. Composition selon les revendications 1 à 6, comprenant des hydrolysats de protéine dans une quantité suffisante pour administrer à un sujet un dosage journalier de 0,01 g par kg de poids corporel à environ 3 g par kg de poids corporel.

8. Composition selon la revendication 5, comprenant des protéines non hydrolysées dans une quantité suffisante pour administrer à un sujet un dosage journalier de 0,01 g par kg de poids corporel à environ 3 g par kg de poids corporel.

9. Composition selon la revendication 1, comprenant des glucides dans une quantité suffisante pour administrer à un sujet un dosage journalier de 0,01 g par kg de poids corporel à environ 7 g par kg de poids corporel.

10. Composition selon l'une quelconque des revendications 1 à 9, destinée à augmenter l'insuline plasmatique.

11. Composition selon l'une quelconque des revendications 1 à 9, destinée à augmenter l'insuline plasmatique du diabète de type 2 ou du pré-diabète.

12. Composition selon l'une quelconque des revendications 1 à 9, destinée à réduire les concentrations du glucose post-prandial dans le sang du diabète de type 2 ou du pré-diabète.

13. Composition selon l'une quelconque des revendications 1 à 9, destinée à augmenter la sécrétion d'insuline post-prandiale dans le sang du diabète de type 2 ou du pré-diabète.
